# FASCICULE DE BREVET EUROPEEN

(11) **EP 0 949 268 B1**
(45) Date de publication et mention de la délivrance du brevet: **10.12.2003**
(21) Numéro de dépôt: 99400843.1
(22) Date de dépôt: 07.04.1999
(51) Int. Cl.: C07H 17/08

(54) **Nouveaux dérivés de la 2-fluoro 3-de[(2,6-didéoxy 3-C-méthyl-3-0-méthyl-alpha-L-ribohexopyranosyl) oxy] 6-0-méthyl 3-oxo érythromycine, leur procédé de préparation et leur application à la synthèse de principes actifs de médicaments**
2-Fluor 3-de (2,6-dideoxy 3-C-methyl-3-0-methyl-alpha-L-ribohexopyranosyl oxy) 6-1-methyl 3-oxo Erythromycin, ihrer Verfahren zur Herstellung und ihrer Verwendung zur Synthese von Arzneimitteln
Derivatives of 2-fluoro 3-de(2,6-dideoxy 3-C-methyl-3-0-methyl-alpha-L-ribohexopyranosyl oxy) 6-1-methyl 3-oxo erythromycin, their process of preparation and application in the synthesis of medicaments

(30) Priorité: 08.04.1998 FR 9804366
(43) Date de publication de la demande: 13.10.1999
(73) Titulaire: Aventis Pharma S.A., 92160 Antony (FR)
(72) Inventeur: Bonnet, Alain, 02400 Chateau Thierry (FR); Gambier, Françoise, 75020 Paris (FR)

(56) Documents cités:
- EP-A- 0 487 411
- EP-A- 0 596 802
- EP-A- 0 799 833
- GRIESGRABER G ET AL: "3-KETO-11,12-CARBAZATE DERIVATIVES OF 6-O-METHYLERYTHROMYCIN A SYNTHESIS AND IN VITRO ACTIVITY" JOURNAL OF ANTIBIOTICS, vol. 49, no. 5, 1 mai 1996, pages 465-477, XP002072221

## Description

La présente invention concerne de nouveaux dérivés de la 2-fluoro 3-de((2,6-didéoxy-3-C-méthyl-3-O-méthyl-α-L-ribo-hexopyrasonyl) oxy) 6-O-méthyl 3-oxo érythromycine, leur procédé de préparation et leur application à la synthèse de principes actifs de médicaments.

L'art antérieur comprend notamment les documents suivants :
- Le document EP-A-0799833 décrit des dérivés 2-Fluorés de 3-oxo érythromycine, comportant en position 11, 12 un groupement carbamate substitué.
- Le document EP-A-487411 décrit quant à lui, entre autres, des dérivés 3-oxo de l'érythromycine, comportant en position 11, 12 un groupement carbonate, mais substitué en 2 soit par un alkyle éventuellement substitué par un groupement amino ou mono ou dialkylamino, soit par un groupement amino ou mono ou dialkylamino.

Enfin, le document EP-A-0596802 décrit des dérivés 3-oxo de l'érythromycine, comportant en position 11, 12 un groupement carbamate substitué, mais ne comportant pas de substituant autre que méthyle en position 2.

L'invention a pour objet les composés de formule (I) ainsi que leurs sels d'addition avec les acides : 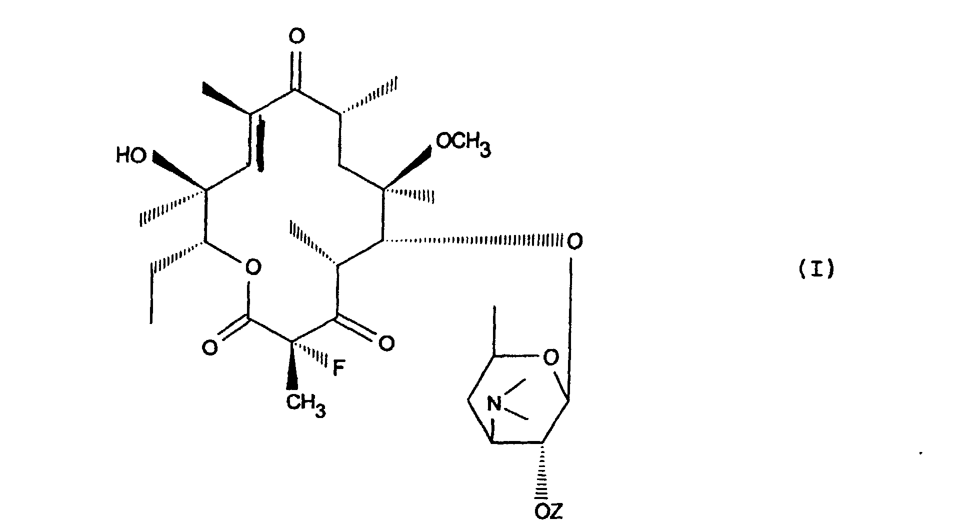 dans lesquels OZ représente un radical hydroxyle libre, estérifié ou éthérifié.

Parmi les sels d'addition avec les acides, on peut citer les sels formés avec les acides fluorhydrique, chlorhydrique, bromhydrique, iodhydrique, acétique, propionique, trifluoroacétique, maléïque, tartrique, méthanesulfonique, benzènesulfonique, p-toluènesulfonique, et spécialement les acides stéarique, éthylsuccinique ou laurylsuccinique. Ces sels sont formés au niveau du groupe diméthylamino de la molécule.

L'invention a notamment pour objet les composés de formule (I), dans lesquels Z représente un atome d'hydrogène, ceux dans lesquels Z représente un radical COCH₃ et ceux dans lesquels Z représente un radical triméthylsilyle.

Parmi les composés préférés de l'invention, on peut citer: tout particulièrement les composés de formule (I) dont la préparation est donnée ci-après dans la partie expérimentale.

L'invention a également pour objet un procédé de préparation, caractérisé en ce que l'on soumet un composé de formule (A) : 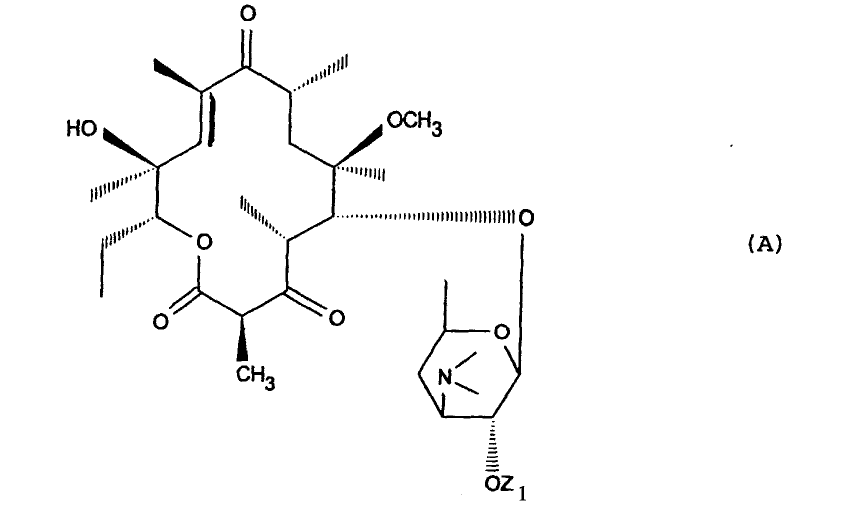 dans laquelle OZ₁ représente un radical hydroxyle estérifié ou éthérifié à l'action d'un agent de fluoration pour obtenir le composé de formule (I) correspondant, puis, si désiré, libère l'hydroxyle en 2', et/ou soumet, si désiré, le composé de formule (I) à l'action d'un acide pour en former le sel au niveau du groupe diméthylamino de la molécule.

Comme agent de fluoration, on peut citer le N-fluoro-Bis (phénylsulfonyl) imide de formule : 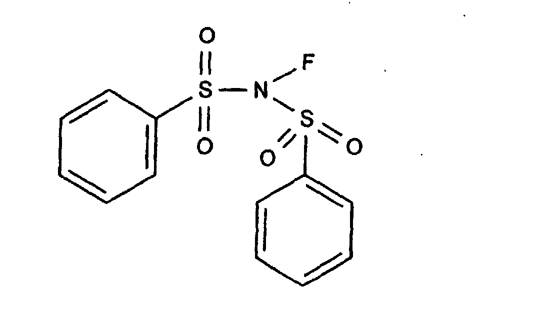 mais également tous les agents de fluoration électrophiles.

Comme agent de libération de l'hydroxyle en 2', on peut citer les bases fortes comme le fluorure de tétrabutylammonium lorsque OZ est un groupement OSi(CH₃)₃ ou les alcools comme le méthanol lorsque OZ est un radical COCH₃.

Les composés de formule (I) trouvent leur utilisation dans la préparation de médicaments ; ils permettent en particulier la préparation des produits du brevet européen 799833.

L'invention a également pour objet l'application des produits de formule (I), caractérisé en ce que l'on soumet un composé de formule (I), à l'action du carbonyldiimidazole pour obtenir le composé de formule (II) : 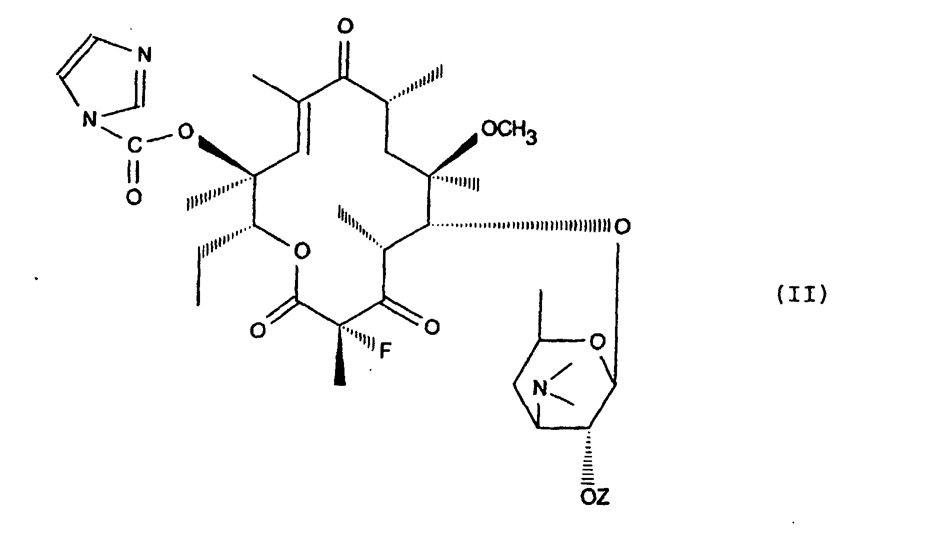 que l'on soumet à l'action d'une amine H₂NR pour obtenir le composé de formule (III) : 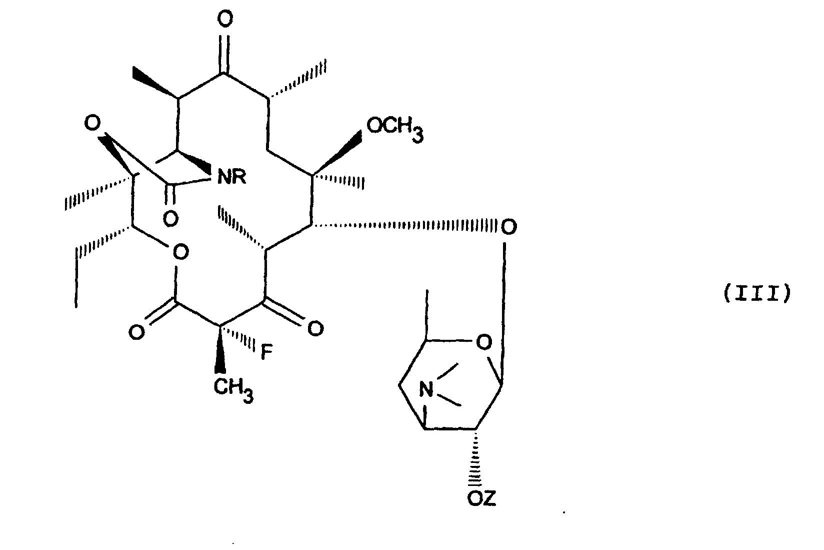 puis si désiré libère l'hydroxyle en 2'.

La partie expérimentale donne un exemple détaillé de l'application des composés de formule (I).

Des composés de formule (III) sont décrits dans la demande européenne EP-0799833.
L'invention a également pour objet à titre de produits chimiques nouveaux les composés de formule (II) définis ci-dessus et notamment
le 2'-acétoxy 2α-fluoro de 12-(oxycarbonylimidazol) 11-déoxy 10,11-didéhydro 3-de[(2,6-didéoxy 3-C-méthyl 3-O-méthyl α-L-ribohexopyranosyl) oxy] 6-O-méthyl 3-oxo érythromycine.

Les exemples suivants illustrent l'invention sans toutefois la limiter.

### EXEMPLE 1 : 2'-triméthylsilyloxy 2α-fluoro de 11-déoxy 10,11-didéhydro 3-de[(2,6-didéoxy-3-C-méthyl-3-O-méthyl α-L-ribohexopyranosyl) oxy] 6-O-méthyl 3-oxo érythromycine.

On ajoute à -12°C sous atmosphère d'argon 1,24 ml d'une solution de terbutylate de potassium dans le THF 0,97M dans une solution renfermant 668 mg de 2'-triméthylsilyloxy de 11-déoxy 10,11-didéhydro 3-de[(2,6-didéoxy-3-C-méthyl-3-O-méthyl α-L-ribohexopyrasonyl) oxy] 6-O-méthyl 3-oxo érythromycine et 6,7 ml de THF anhydre. On agite 5 minutes et ajoute 378 mg de N-fluoro dibenzènesulfonimide. On agite 10 minutes à -12°C et laisse revenir à la température ambiante pendant 1 heure 30 minutes. On effectue les opérations d'isolation et purification et obtient 695 mg du produit recherché.
RMN 250 MHz CDCl₃ : H₁₁ (s) : 6,42 ; H₁₃ (dd) : 4,85 ; 6-OMe : 2,55 (s), N(Me)₂ : 2,12 (s) ; CH₃-C-F (d) : 1,60 J=22Hz.

### Préparation du produit de départ de l'exemple 1.

### Stade A : 11-déoxy 10,11-didéhydro 3-de[(2,6-didéoxy-3-C-méthyl 3-O-méthyl α-L-ribohexopyranosyl) oxy] 6-O-méthyl 3-oxo érythromycine.

On agite pendant 44 heures un mélange de 8,722 g de 2'-acétoxy de 11-déoxy 10,11-didéhydro 3-de[(2,6-didéoxy-3-C-méthyl-3-0-méthyl α-L-ribohexopyranosyl) oxy] 6-O-méthyl 3-oxo érythromycine (EP 596802) et 350 ml de méthanol anhydre. On évapore, reprend au chlorure de méthylène, sèche et obtient 8,794 g du produit recherché.
RMN 250 MHz CDCl₃ : H₁₁ (s) : 6,64 ; H₁₃ (dd) : 4,99 ; H'₁ : 4,25 (d) ; 6-OMe (s) : 2,87, 10 Me (s) : 1, 96 (s) ; N(Me)₂ (s) : 2,25.

### Stade B : 2'-triméthylsilyloxy de 11-déoxy 10,11-didéhydro 3-de[(2,6-didéoxy-3-C-méthyl-3-O-méthyl α-L-ribohexopyranosyl) oxy] 6-O-méthyl 3-oxo érythromycine.

On agite à température ambiante pendant 4 jours un mélange renfermant 3,08 g du produit du stade précédent, 340 mg d'imidazole, 32 ml de THF anhydre et 1,06 ml d'hexaméthyl-disilylazane. On évapore à sec, reprend avec un mélange de 60 ml de chlorure de méthylène et de 60 ml de phosphate acide de sodium 0,5 M. On maintient le mélange réactionnel sous agitation pendant 15 minutes, décante, extrait au chlorure de méthylène, sèche et évapore à sec. On obtient 3,345 g du produit recherché.
RMN 250 MHz CDCl₃ : H₁₁ : 6, 61 (s) ; H₁₃ (dd) : 4,92 ; 60Me (s) : 2,85 ; N(Me)₂ : 2,15 (s) : SiMe₃ (s) : 0, 02.

### EXEMPLE 2 : 2'-acétoxy 2α-fluoro de 11-déoxy 10,11-didéhydro 3-de[(2,6-didéoxy 3-C-méthyl 3-O-méthyl α-L-ribohexopyrasonyl) oxy] 6-O-méthyl 3-oxo érythromycine.

On ajoute à -8°C, 2,5 ml d'une solution de terbutylate de potassium dans le THF (0,97M) dans une suspension renfermant 1,224 g de 2'-acétoxy de 11-déoxy 10,11-didéhydro 3-de[(2,6-didéoxy 3-C-méthyl 3-O-méthyl α-L-ribohexopyranosyl) oxy] 6-O-méthyl 3-oxo érythromycine (EP 596802) et 10 ml de THF anhydre. On ajoute 756 mg de N-fluorodibenzènesulfonimide. On agite pendant 1 heure à -5°C puis ajoute 10 ml d'eau bicarbonatée saturée et 10 ml d'acétate d'éthyle, agite 10 minutes à la température ambiante. On filtre, rince, décante, lave à l'eau, réextrait à l'acétate d'éthyle, lave sèche et filtre. On chromatographie sur silice en éluant avec un mélange chlorure de méthylène-méthanol à 8% d'ammoniaque concentrée 95-5. On obtient 623 mg de produit recherché.
RMN : H₁₁ : 6,47 (s) ; H₁₃ : 5, 03 (dd) ; 6-OMe : 2, 66 (s) ; N-Me₂ : 2, 25 (s) : CH₃-C-F : 1,75 (d) J = 21,5 Hz.

### Application: 11,12-didéoxy 3-de[(2,6-didéoxy 3-C-méthyl 3-O-méthyl-α-L-ribohexopyrasonyl) oxy] 2α-fluoro 6-O-méthyl 3-oxo 12,11-[oxycarbonyl [4-[4-(3-pyridinyl) 1H-imidazol-1-yl] butyl] imino] érythromycine A.

### Stade A : 2α-fluoro de 11-déoxy 10,11-didéhydro 3-de[(2,6-didéoxy 3-C-méthyl 3-O-méthyl α-L-ribohexopyranosyl) oxy] 6-O-méthyl 3-oxo érythromycine.

On agite pendant 3 heures 30 minutes un mélange de 5,476 g de produit de l'exemple 1, 50 ml de THF et 11,2 ml de fluorure de tétrabutylammonium 1M dans le THF. On évapore le solvant et ajoute 37 ml d'acétate d'éthyle, 37 ml d'eau et 7,5 ml d'ammoniaque à 20%. On agite 10 minutes, décante, extrait à l'acétate d'éthyle, sèche, filtre et concentre à sec le filtrat. On chromatographie le produit obtenu sur silice en éluant avec le mélange CH₂Cl₂-MeOH ammoniaqué 99-1, puis 98-2, 97-3, 96-4, 95-5. On obtient 2,452 g de produit recherché.

### Stade B : 2'-acétoxy 2α-fluoro de 11-déoxy 10,11-didéhydro 3-de[(2,6-didéoxy 3-O-méthyl α-L-ribohexopyrasonyl) oxy] 6-0-méthyl 3-oxo érythromycine.

On maintient sous agitation pendant 3 heures 1,02 g de produit du stade A, 10 ml de chlorure de méthylène et 241 µl d'anhydride acétique. On évapore et ajoute 10 ml d'eau et 10 ml d'acétate d'éthyle. On laisse 1 heure à la température ambiante sous agitation, décante, sèche et évapore. On obtient 1,01 g de produit recherché.
CCM SiO₂ dichlorométhane 95 - MeOH 5 ammoniaqué, rf : 0,14. RMN 250 MHz CDCl₃ : H₁₁ (s) : 6,47 ; H'₂ (q) : 4,75 ; N(Me)₂ (s) : 2,22 ; CH₃-CO-O (s) : 2,05 ; CH₃-C-F (d) : 1,75 ; J = 22 Hz.

### Stade C : 2'-acétoxy 2α-fluoro de 12-(oxycarbonylimidazol) 11-déoxy 10,11-didéhydro 3-de[(2,6-didéoxy 3-C-méthyl 3-0-méthyl α-L-ribohexopyranosyl) oxy] 6-O-méthyl 3-oxo érythromycine.

On ajoute à 0°C 0,388 g de carbonyldiimidazole et 24 µl de DBU dans une solution renfermant 1,01 g du produit du stade précédent et 10 ml de THF anhydre. On évapore le THF et ajoute 10 ml d'eau et 10 ml d'acétate d'éthyle. On maintient le mélange réactionnel sous agitation pendant 10 minutes, extrait, sèche et évapore. On obtient 0,902 g de produit recherché brut que l'on chromatographie en éluant avec un mélange acétate d'êthyle-triéthylamine 96-4. On obtient 0,573 g de produit recherché.
RMN (CDCl₃) : H₁₁ : 6, 69 (s) ; H₁₃ : 5,55 (dd) ; 6-OMe : 2,62 (s) ; N-Me₂ : 2, 25 (s) : 10-Me : 1, 90 (s).

### Stade D : 2'-acétoxy 11,12-didéoxy 3-de[(2,6-didéoxy 3-C-méthyl 3-O-méthyl α-L-ribohexopyrasonyl) oxy] 2α-fluoro 6-O-méthyl 3-oxo 12,11-[oxycarbonyl [4-[4-(3-pyridinyl) 1H-imidazol-1-yl] butyl] imino] érythromycine A.

On ajoute à 0°C 211 mg de 4-(3-pyridinyl) 1H-imidazol-1-butylamine, 573 mg de produit du stade précédent et 5 ml de THF anhydre. On ajoute 19 µl de DBU. On maintient le mélange réactionnel au réfrigérateur pendant une nuit. On évapore et ajoute 10 ml d'acétate d'éthyle et 10 ml d'eau. On agite 10 minutes , extrait, sèche et évapore. On obtient 0,545 g de produit recherché brut que l'on utilise tel quel pour le stade suivant.

### Stade E : 11,12-didéoxy 3-de[(2,6-didéoxy 3-C-méthyl 3-O-méthyl-α-L-ribohexopyranosyl) oxy] 2α-fluoro 6-O-méthyl 3-oxo 12,11-[oxycarbonyl [4-[4-(3-pyridinyl) 1H-imidazol-1-yl] butyl] imino] érythromycine A.

On reprend le produit précédent dans le méthanol. On maintient le mélange réactionnel sous agitation à la température ambiante pendant 24 heures. On chromatographie sur silice le produit obtenu en éluant avec le mélange acétate d'éthyle-triéthylamine 96-4. On évapore. On obtient 189 mg du produit recherché.

## Revendications

1. Les composés de formule (I) ainsi que leurs sels d'addition avec les acides : 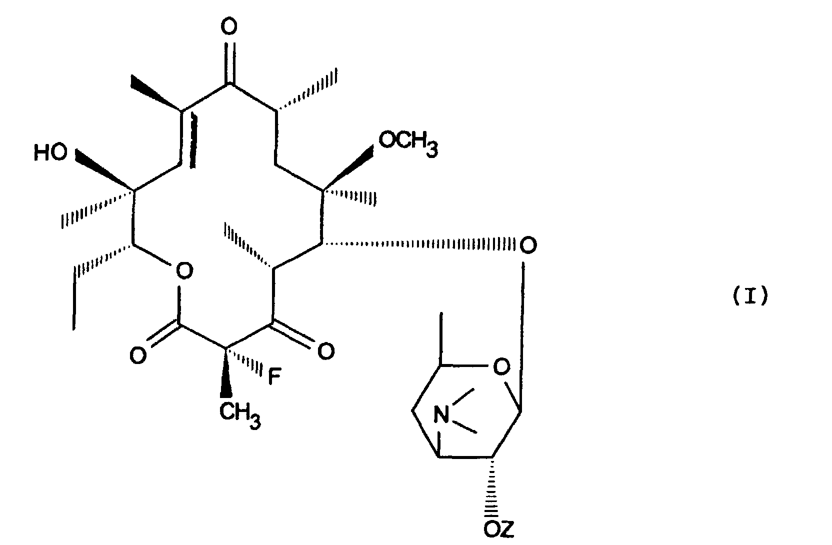 dans lesquels OZ représente un radical hydroxyle libre, estérifié ou éthérifié.

2. Les composés de formule (I) définis à la revendication 1, dans lesquels Z représente un atome d'hydrogène.

3. Les composés de formule (I) définis à la revendication 1, dans lesquels Z représente un radical COCH₃.

4. Les composés de formule (I) définis à la revendication 1, dans lesquels Z représente un radical triméthylsilyle.

5. Procédé de préparation des composés de formule (I), **caractérisé en ce que** l'on soumet un composé de formule (A) : 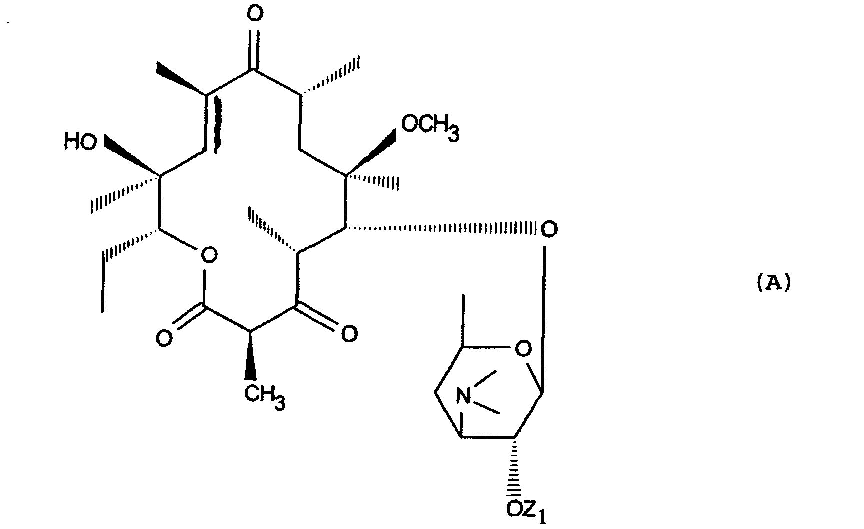 dans laquelle OZ₁ représente un radical hydroxyle estérifié ou éthérifié, l'action d'un agent de fluoration pour obtenir le composé de formule (I) correspondant, puis, si désiré, libère l'hydroxyle en 2', et/ou soumet, si désiré, le composé de formule (I) à l'action d'un acide pour en former le sel au niveau du groupe diméthylamino de la molécule.

6. Application des composés de formule (I) définis à la revendication 5, **caractérisée en ce que** l'on soumet un composé de formule (I), à l'action du carbonyldiimidazole pour obtenir le composé de formule (II) : 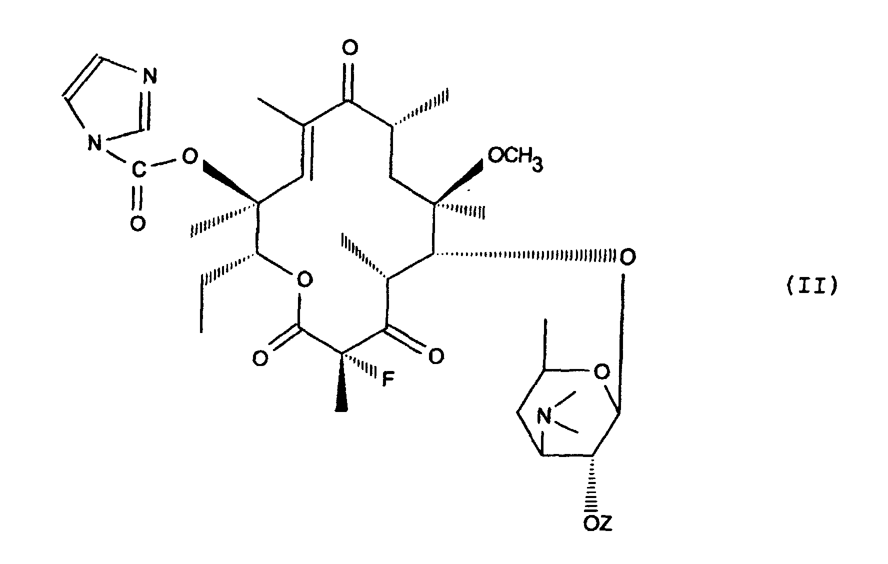 que l'on soumet à l'action d'une amine H₂NR pour obtenir le composé de formule (III) : 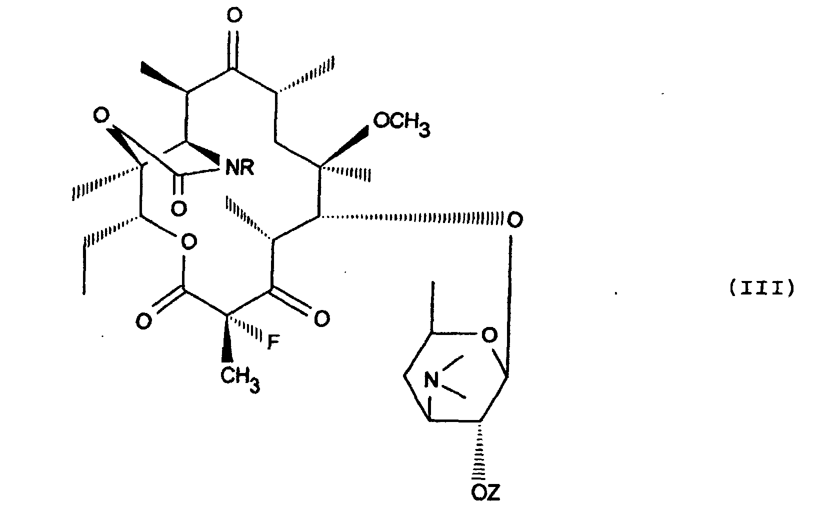 puis si désiré libère l'hydroxyle en 2'.

7. A titre de produits chimiques, les composés de formule (II) définis à la revendication 6.

## Claims

1. The compounds of formula (I) as well as their addition salts with acids: 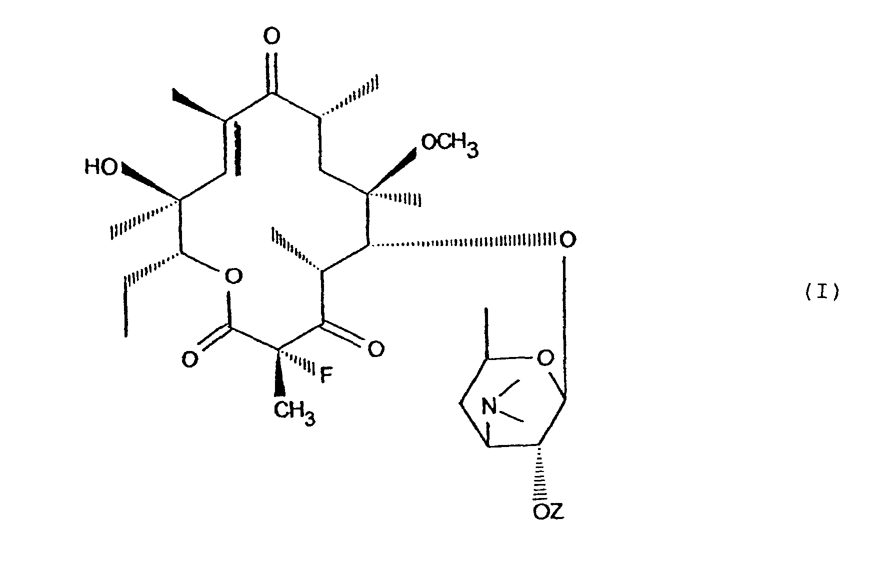 in which OZ represents a free, esterified or etherified hydroxyl radical.

2. The compounds of formula (I) defined in claim 1, in which Z represents a hydrogen atom.

3. The compounds of formula (I) defined in claim 1, in which Z represents a COCH₃ radical.

4. The compounds of formula (I) defined in claim 1, in which Z represents a trimethylsilyl radical.

5. Process for the preparation of the compounds of formula (I), **characterized in that** a compound of formula (A): 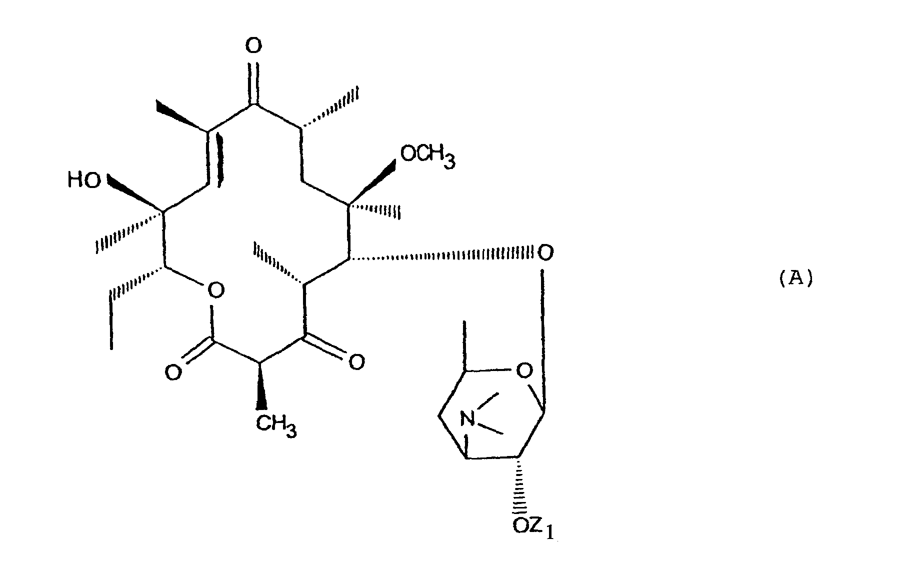 in which OZ₁ represents an esterified or etherified hydroxyl radical, is subjected to the action of a fluorination agent in order to obtain the corresponding compound of formula (I), then, if desired, the hydroxyl in position 2' is released, and/or, if desired, the compound of formula (I) is subjected to the action of an acid in order to form the salt at the level of the dimethylamino group of the molecule.

6. Use of the compounds of formula (I) defined in claim 5, **characterized in that** a compound of formula (I) is subjected to the action of carbonyldiimidazole in order to obtain the compound of formula (II): 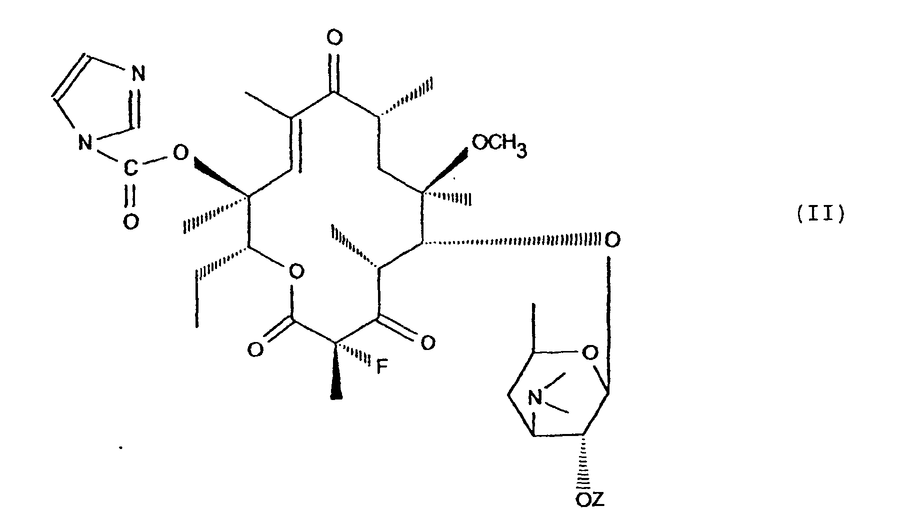 which is subjected to the action of an H₂NR amine in order to obtain the compound of formula (III): 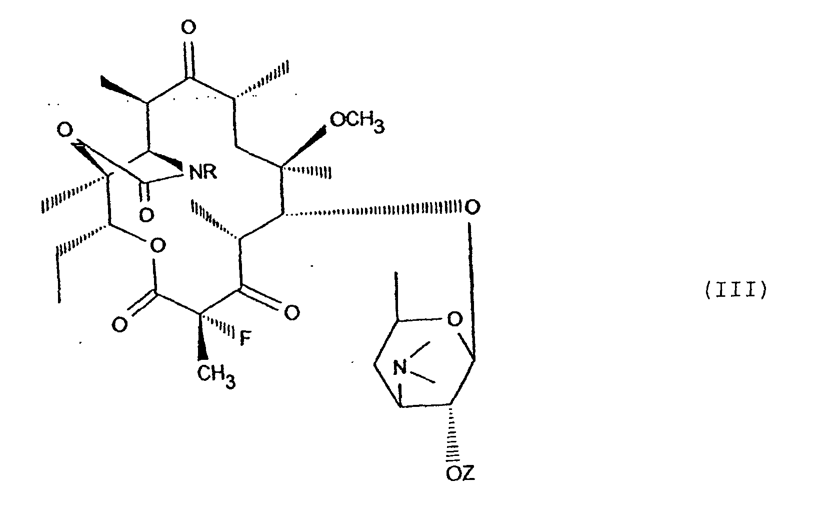 then if desired the hydroxyl in position 2' is released.

7. As chemical products, the compounds of formula (II) defined in claim 6.

## Patentansprüche

1. Verbindungen der Formel (I) sowie ihre Additionssalze mit Säuren: 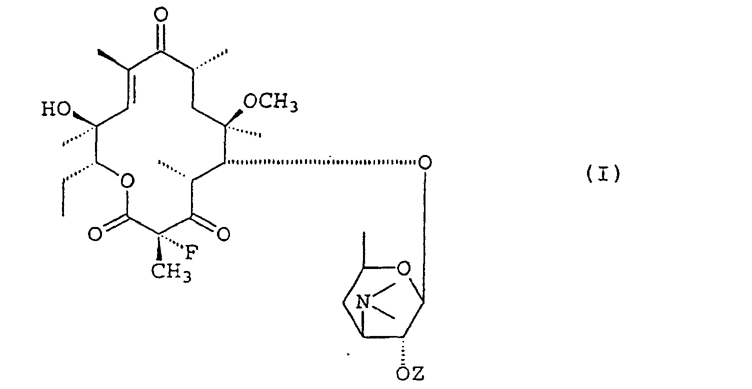 in der
OZ einen freien, veresterten oder veretherten Rest Hydroxyl darstellt.

2. Verbindungen der Formel (I) wie in Anspruch 1 definiert, worin Z ein Wasserstoffatom darstellt.

3. Verbindungen der Formel (I) wie in Anspruch 1 definiert, worin Z einen Rest COCH₃ darstellt.

4. Verbindungen der Formel (I) wie in Anspruch 1 definiert, worin Z einen Rest Trimethylsilyl darstellt.

5. Verfahren zur Herstellung der Verbindungen der Formel (I), **dadurch gekennzeichnet, daß** man eine Verbindung der Formel (A) 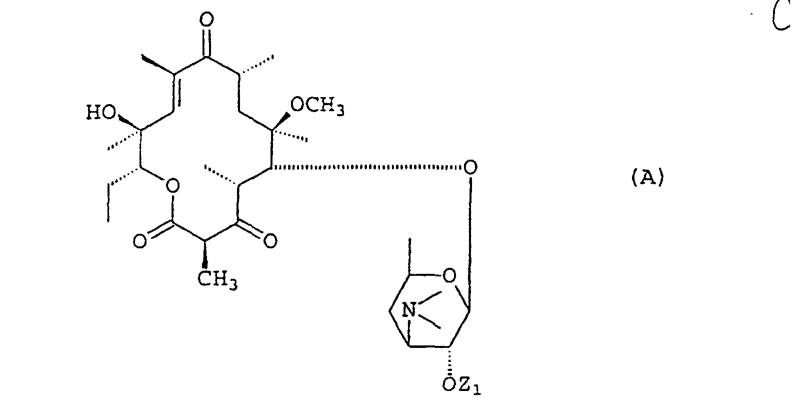 in der OZ₁ einen veresterten oder veretherten Rest Hydroxyl darstellt, der Einwirkung eines Fluorierungsmittels unterzieht, um die entsprechende Verbindung der Formel (I) zu erhalten, und man anschließend, wenn erwünscht, das Hydroxyl in 2' freisetzt, und/ oder, wenn erwünscht, die Verbindung der Formel (I) der Einwirkung einer Säure unterzieht, um daraus das Salz im Bereich der Gruppe Dimethylamino des Moleküls zu bilden.

6. Verwendung der Verbindungen der Formel (I) wie in Anspruch 5 definiert, **dadurch gekennzeichnet, daß** man eine Verbindung der Formel (I) der Einwirkung von Carbonyldiimidazol unterzieht, um die Verbindung der Formel (II) 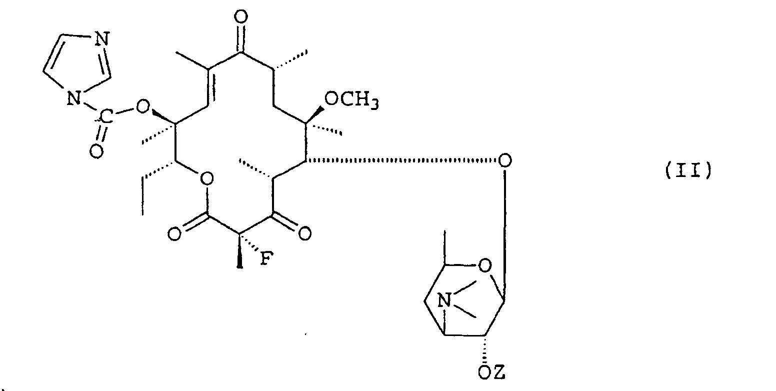 zu erhalten,
die man dann der Einwirkung eines Amins H₂NR unterzieht, um die Verbindung der Formel (III) 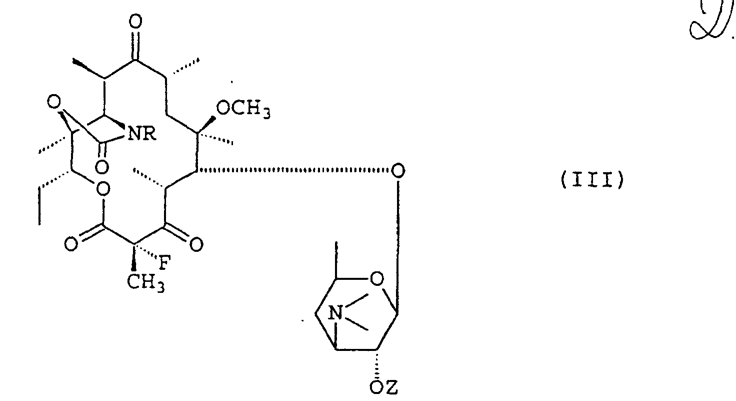 zu erhalten,
bei der man anschließend, wenn erwünscht, das Hydroxyl in 2' freisetzt.

7. Als chemische Produkte die Verbindungen der Formel (II) wie in Anspruch 6 definiert.
